# EUROPEAN PATENT APPLICATION

(11) **EP 3 220 369 A1**
(43) Date of publication of application: **20.09.2017**
(21) Application number: 17154495.0
(22) Date of filing: 02.02.2017
(51) Int. Cl.: G08B 25/01, G08B 25/10, A61B 5/00, A61B 5/01, A61B 5/0205, A61B 5/024, A61B 5/053, G01S 19/17

(54) **MONITORING USER BIOMETRIC PARAMETERS WITH NANOTECHNOLOGY IN PERSONAL LOCATOR BEACON**

(30) Priority: 15.03.2016 IN 201611009035; 29.09.2016 US 201615279481
(71) Applicant: Hand Held Products, Inc., Fort Mill, SC 29707 (US)
(72) Inventor: RAMAN, Kannan, Morris Plains, NJ 07950 (US); THUPALLI, Sai Bhanu Prakash, Morris Plains, NJ 07950 (US)
(74) Representative: Houghton, Mark Phillip

(57) **Abstract**

A personal locator beacon system has a personal locator beacon and a biometrics monitor. The personal locator beacon includes a first microprocessor, a first global positioning subsystem coupled to the first microprocessor, a first low energy transceiver coupled to the first microprocessor, and a first low energy antennae coupled to the first low energy transceiver. The biometrics monitor includes a second microprocessor, a second low energy transceiver coupled to the second microprocessor, a second low energy antennae coupled to the second low energy transceiver, and one or more nanosensors.

## Description

### CROSS-REFERENCE TO RELATED APPLICATION

The present application claims the benefit of Indian Patent Application No. 201611009035 for *Monitoring User Biometric Parameters with Nanotechnology in Personal Locator Beacon* filed March 15, 2016, which is hereby incorporated by reference in its entirety.

### FIELD OF THE INVENTION

The invention is generally related to personal locator beacons, and, more specifically, to personal locator beacon systems that monitor user biometric parameters.

### BACKGROUND

Personal tracker beacons are devices that track a user's geographic details, such as the user's latitude and longitude, using GPS satellite data. These beacons generally have a wireless transmitter that can be activated in life-threatening emergency situations to broadcast the user's geographic location to emergency personnel. The wireless transmitter can broadcast on a number of different frequencies, such as over local cellular networks, legacy analogue signal bands of 121.5 MHz or 243 MHz, or over the internationally designated 406 MHz digital radio-frequency band. The 406 MHz band has been designated an emergency band under the International Cospas-Sarsat Programme, which is an intergovernmental cooperative of 43 countries and agencies that maintains a network of satellites and ground facilities to receive distress signals from 406-MHz beacons and route the alerts to the proper authorities in more than 200 countries and territories. While geographical location information is critical in locating individuals in emergency situations, health data is not provided along with the location data. Thus, Search and Rescue teams responding to an emergency do not know the physical condition of the individual in distress, and must carry a general emergency kit that addresses a wide variety of situations. If the personal tracker beacon was equipped to provide health data on the individual in distress, Search and Rescue teams could tailor their emergency kits to better address the needs of the individual.

### SUMMARY

In an aspect of the invention, a personal locator beacon system comprises: a personal locator beacon comprising a first microprocessor, a first global positioning subsystem coupled to the first microprocessor, a first low energy transceiver coupled to the first microprocessor, and a first low energy antennae coupled to the first low energy transceiver; and a biometrics monitor comprising a second microprocessor, a second low energy transceiver coupled to the second microprocessor, a second low energy antennae coupled to the second low energy transceiver, and one or more nanosensors.

In an embodiment, the first low energy transceiver, the first low energy antennae, the second low energy transceiver, and the second low energy antennae are low energy Bluetooth components.

In an embodiment, the personal locator beacon is communicatively coupled to the biometrics monitor through the first low energy transceiver and antennae and the second low energy transceiver and antennae.

In an embodiment, the nanosensor is a bioimpedance sensor configured to measure one or more of a user heart rate, respiration level, or hydration level.

In another embodiment, the nanosensor is an optical heart rate sensor.

In another embodiment, the nanosensor is a galvanic skin response sensor configured to monitor user sweat levels.

In another embodiment, the nanosensor is an accelerometer configured to count user steps or record sudden changes in movement.

In yet another embodiment, the nanosensor is a gyroscope configured to measure orientation of a user.

In yet another embodiment, the nanosensor is a thermometer configured to monitor user body temperature.

In another embodiment, the nanosensor is a radiation sensor configured to measure user radiation exposure.

In yet another embodiment, the radiation sensor measures user radiation exposure to ultra-violet, high-energy beta, gamma, x-ray frequencies, or any combination thereof.

In an embodiment, the nanosensor comprises a bioimpedance sensor, an optical heart rate sensor, a galvanic skin response sensor, an accelerometer, a gyroscope, a thermometer, ultra-violet radiation sensor, or any combination thereof.

In an embodiment, the biometrics monitor comprises a second GPS subsystem coupled to the second microprocessor.

In another embodiment, each of the first and second GPS subsystems comprise: a GPS receiver, a GPS antenna, and a GPS static memory communicatively coupled to the GPS receiver and configured to store: positioning information, time stamps associated with the positioning information, route information, speed of travel, or any combination thereof.

In an embodiment, the personal locator beacon comprising a beacon static memory communicatively coupled to the first microprocessor, being configured to receive and store nanosensor data for a period of configurable days.

In an embodiment, the biometrics monitor is a wearable device comprising a glove, a wristband, a necklace, a headband, a hat, smartphone, smartwatch or a chest strap.

In an embodiment, the biometrics monitor is a portable device carryable in a beltpack, backpack, or other external container.

In another aspect of the invention, a method of monitoring user biometric parameters in a personal locator beacon system, comprises: providing a personal locator beacon wirelessly coupled to a wearable biometrics monitor comprising one or more nanosensors; detecting a user biometric parameter of a user by the nanosensor; communicating the user biometric parameter from the biometric monitor to the personal locator beacon; and broadcasting from the personal locator beacon a distress signal comprising geographical location information and the user biometric parameter.

In an embodiment, the personal locator beacon comprises a GPS subsystem having: a GPS receiver, a GPS antenna, and a static memory coupled to the GPS receiver and configured to store positioning information and associated time stamps.

In another embodiment, the nanosensor comprises: a bioimpedance sensor configured to measure one or more user biometric parameters of a user heart rate, respiration level, or hydration level; an optical heart rate sensor configured to measure a user biometric parameter of a user heart rate; a galvanic skin response sensor configured to monitor a user biometric parameter of user sweat level; an accelerometer configured to measure a user biometric parameter of user steps or sudden changes in user movement; a gyroscope configured to measure a user biometric parameter of a user orientation; a thermometer configured to monitor a user biometric parameter of user body temperature; a radiation sensor configured to monitor user radiation exposure levels; or any combination thereof.

In another embodiment, the biometric monitor is a wearable device comprising a glove, a wristband, a necklace, a headband, a hat, smartphone, smartwatch, or a chest strap.

In an embodiment, the personal locator beacon is wirelessly coupled to the wearable biometric monitor using Bluetooth low energy.

In an embodiment, the user biometric parameters from the biometric monitor are communicated to the personal locator beacon at configured intervals.

In another embodiment, the configured intervals are event triggered intervals, predetermined fixed intervals, profile based intervals, or any combination thereof.

In an embodiment, the personal locator beacon is a Cospas-Sarsat distress beacon or a vehicle satcom relay.

### BRIEF DESCRIPTION OF THE DRAWINGS

The invention will now be described by way of example, with reference to that accompanying Figures, of which:
Figure 1 is a perspective view of a personal locator beacon system;
Figure 2 is a schematic diagram of the personal locator beacon;
Figure 3 is a schematic diagram of the biometrics monitor;
Figure 4 is a perspective view of a biometrics monitor; and
Figure 5 is a flow diagram of a method of monitoring biometrics parameters in the personal locator beacon system.

### DETAILED DESCRIPTION

All Cospas-Sarsat beacons are subject to the same radio-frequency specifications, but the beacons can be fashioned into a variety of mechanical structures. Additionally, the beacons can have a variety of disparate activation methods, the details of which are often tailored to different applications, and named accordingly: a) Emergency Position Indicating Radio Beacon ("EPIRB") for marine use; b) Emergency Locator Transmitter ("ELT") for aviation use; and c) Personal Locator Beacon ("PLB") for personal and/or terrestrial use. For the purpose of this invention, the term "PLB" will be generally used, along with "Locator Beacon", or "Beacon". Thus, "PLB" should not be interpreted in a restricted sense unless expressly stated, and will be understood to refer to any type of radio locator beacon (not necessarily restricted only to "personal").

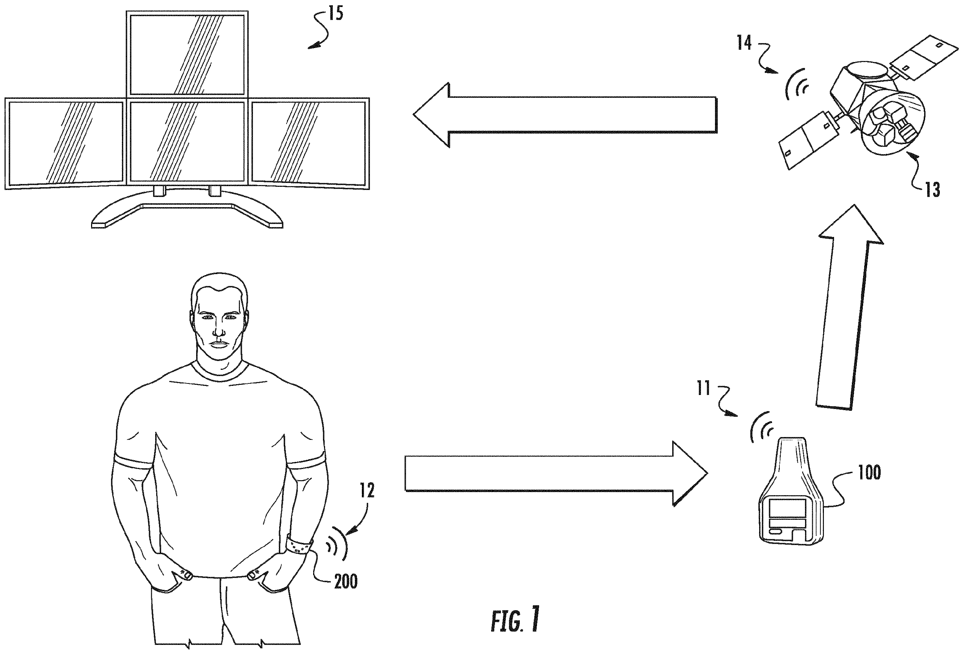

In an embodiment shown in Figure 1, a personal locator beacon system 1 includes a personal locator beacon 100 and biometrics monitor 200.

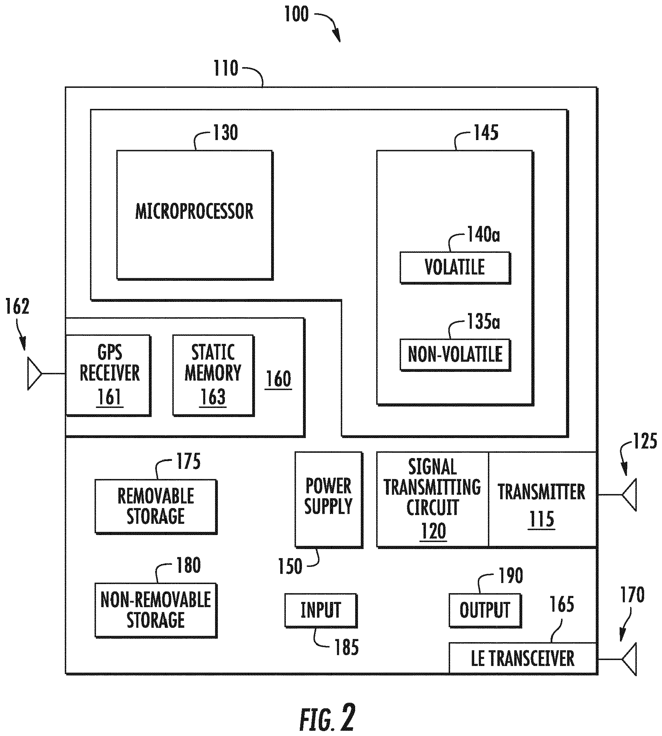

In an embodiment shown in Figure 2, the personal locator beacon 100 includes a housing 110 having one or more of a radio frequency transmitter 115, signal transmitting circuit 120, first radio frequency antenna 125, first microprocessor 130, first memory 145, power supply 150, first global positioning system ("GPS") subsystem 160, first low energy transceiver 165, and a first low energy antenna 170.

As shown in the embodiment of Figure 2, the housing 110 houses the various components of the personal locator beacon 100, and can be any variety of shapes or sizes, depending on the application (e.g. a user worn, vehicle mounted, etc.). The housing 110 can be made of a thermoset or thermoplastic material, metal, composite material, or any combination thereof.

The radio frequency transmitter 115 is electrically connected to the first radio frequency antenna 125 and the signal transmitting circuit 120. The signal transmitting circuit 120 is electrically connected to the first microprocessor 130. The first microprocessor 130 sends radio transmission instructions to the radio frequency transmitter 115 via the signal transmitting circuit 120. The radio frequency transmitter 115 sends signals to the first radio frequency antenna 125 to be transmitted by the first radio frequency antenna 125 on domestic or internationally recognized radio-frequency distress bands. In an embodiment, the first radio frequency antenna 125 transmits signals at approximately 406 MHz. In another embodiment, the first radio frequency antenna 125 transmits signals at approximately 121.5 MHz. In another embodiment, the first radio frequency antenna 125 transmits signals at approximately 243 MHz. In another embodiment, the first radio frequency antenna 125 transmits signals at a frequency corresponding to local cellular phone networks, such as 700MHz, 800MHz, 850MHz, 1700MHz, 1900MHz, or any other commonly used cellular phone frequencies used by cellular phone networks. In an embodiment (not shown), the personal locator beacon 100 includes one or more microprocessors 130 connected to transmitting signal circuit. Those of ordinary skill in the art would appreciate that in an embodiment, the radio frequency transmitter 115 can transmit on two or more of the above described frequency bands. In an embodiment, the radio frequency transmitter 115 is a transceiver.

In an embodiment shown in Figure 2, first memory 145 can include volatile memory (e.g. RAM) 140a and non-volatile memory 135a (e.g. ROM) electrically connected to first microprocessor 130. Personal locator beacon 100 can include - or have access to a computing environment that includes - a variety of computer-readable media, such as the volatile memory 140a and non-volatile memory 135a, a removable storage 175, and non-removable storage 180. First memory 145 storage includes the random access memory

(RAM) 140 and read only memory (ROM) 135, as well as erasable programmable read-only memory (EPROM), electrically erasable programmable read-only memory (EEPROM), flash memory or other memory technologies, compact disc read-only memory (CD ROM), Digital Versatile Disks (DVD) or other optical disk storage, magnetic cassettes, magnetic tape, magnetic disk storage or other magnetic storage devices, or any other medium capable of storing computer-readable instructions.

Personal locator beacon 100 can include or have access to a computing environment that includes input 185 and/or output 190. Output 190 can include a display device, such as a touchscreen, that also can serve as an input device. The input 185 can include one or more of a touchscreen, touchpad, mouse, keyboard, camera, one or more device-specific buttons, one or more sensors integrated within or coupled via wired or wireless data connections to the personal locator beacon 100, and other input devices.

Computer-readable instructions are stored on a computer-readable medium, such as the first memory 145, and are executable by the first microprocessor 130.

In an embodiment shown in Figure 2, a power supply 150 provides power to the components of the personal locator beacon 100. An example of the power supply 150 is a battery, although other sources of power could also provide the requisite power to the components of the personal locator beacon 100.

In an embodiment shown in Figure 2, the first GPS subsystem 160 includes a GPS receiver 161, GPS antenna 162, and a GPS static memory 163. The GPS receiver 161 is electrically connected to the first microprocessor 130, GPS antenna 162, and GPS static memory 163. The GPS receiver 161 receives and processes signals from positional satellites via the GPS antenna 162. Generally, the processed signals are positioning information and associated time stamps. In turn, the GPS receiver 161 sends the processed signals to the GPS static memory 163, which stores current and/or past positioning information and associated time stamps. The GPS static memory 163 is electrically connected to the first microprocessor 130, and the stored current and/or past positioning information and associated time stamps can be accessed by the first microprocessor 130, which in turn, can send this information for storage in memory 135,140,145, and/or can be sent to signal transmitting circuit 120 upon PLB 100 activation. Additionally, in an embodiment the GPS static memory 163 can store route information and speed of travel for a user based on the current and past positioning information and associated time stamps, and this information can also be accessed by the first microprocessor 130

As shown in the embodiment of Figure 2, the first low energy transceiver 165 is electrically connected to the first low energy antenna 170 and to the first microprocessor 130. In an embodiment the first low energy transceiver 165 and first low energy antenna 170 are low energy Bluetooth components. The first low energy transceiver 165 receives signals detected by the first low energy antenna 170 and sends the signals to the first microprocessor 130. Additionally the first low energy transceiver 165 receives signals from the first microprocessor 130, and broadcasts those signals via the first low energy antenna 170.

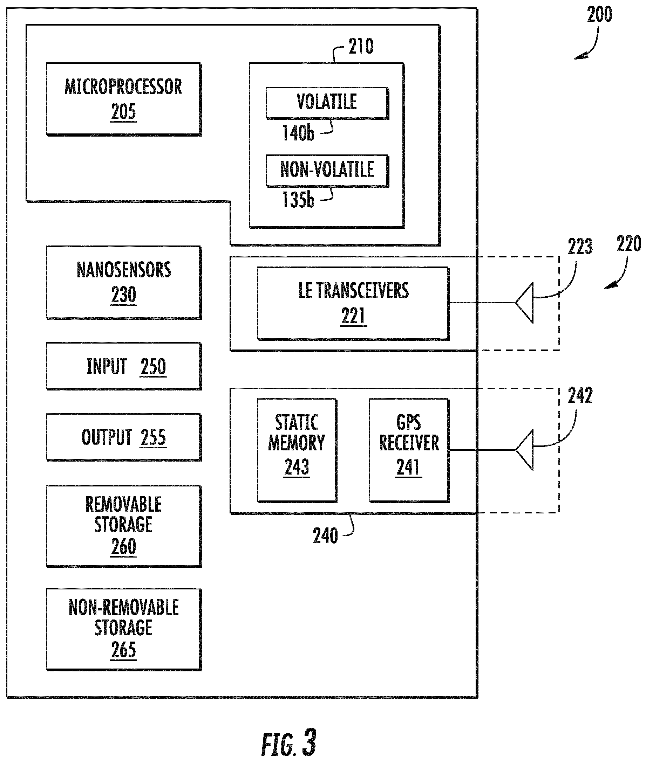

In an embodiment shown in Figure 3, the biometrics monitor 200 is a user-wearable device that includes a second microprocessor 205, second memory 210, wireless communication system 220, and one or more nanosensors 230. The biometrics monitor 200 can be fashioned in many forms, including a glove, wristband, necklace, headband, hat, chest strap, or any other wearable device. In an embodiment, the biometrics monitor 200 is a smartphone, smartwatch, or fitness band having a one or more biometric nanosensors 230.

The term biometric nanosensor referred to herein is any biometric sensor sufficiently small in size and weight to be suitable for personal use. By way of non-limiting example, biometric nanosensors can include, but are not limited to, biometric sensors positioned in devices that can be carried in a backpack, beltpack/fannypack, or similar user-carryable housing; biometric sensors in wearable devices, as discussed further herein; and biometric sensors that may be injected, implanted, or otherwise carried inside the body. In addition, the biometric nanosensors can be contained in or associated with devices having significant functionality in addition to monitoring or measurement of biometrics, such as smartphones, smartwatches, or in devices whose primary or sole functionality consists of monitoring or measurement of biometrics, such as commercially available fitness bands.

The second microprocessor 205 is electrically connected to the second memory 210, wireless communication system 220, and to one or more of the nanosensors 230.

The second microprocessor 205 is substantially similar in function and structure to the first microprocessor 130, and the second memory 210 is substantially similar to the first memory 145.

In an embodiment shown in Figure 3, second memory 210 can include volatile memory (e.g. RAM) 140b and non-volatile memory 135b (e.g. ROM) electrically connected to second microprocessor 205. Biometrics monitor 200 can include - or have access to a computing environment that includes - a variety of computer-readable media, such as the volatile memory 140b and non-volatile memory 135b, a removable storage 260, and non-removable storage 265. Second memory 210 storage includes the random access memory (RAM) 140b and read only memory (ROM) 135b, as well as erasable programmable read-only memory (EPROM), electrically erasable programmable read-only memory (EEPROM), flash memory (e.g. solid state drives), or other memory technologies,

Biometrics monitor 200 can include or have access to a computing environment that includes input 250 and/or output 255. Output 255 can include a display device, such as a touchscreen, that also can serve as an input device. The input 250 can include one or more of a touchscreen, touchpad, mouse, keyboard, camera, one or more device-specific buttons, one or more sensors integrated within or coupled via wired or wireless data connections to the biometrics monitor 200, and other input devices.

Computer-readable instructions are stored on a computer-readable medium such as the second memory 210, and are executable by the second microprocessor 205.

The wireless communication system 220 includes a second low energy (LE) transceiver 221 communicatively coupled to the second microprocessor 205, and a second low energy (LE) antenna 223 electrically coupled to the second low energy (LE) transceiver 221. In an embodiment, the second low energy transceiver 221 and second low energy antenna 223 are low energy Bluetooth components. The second low energy transceiver 221 receives signals from the second low energy antenna 223 and sends the signals to the second microprocessor 205. Additionally the second low energy transceiver 221 receives signals from the second microprocessor 205, and broadcasts those signals using the second low energy antenna 223.

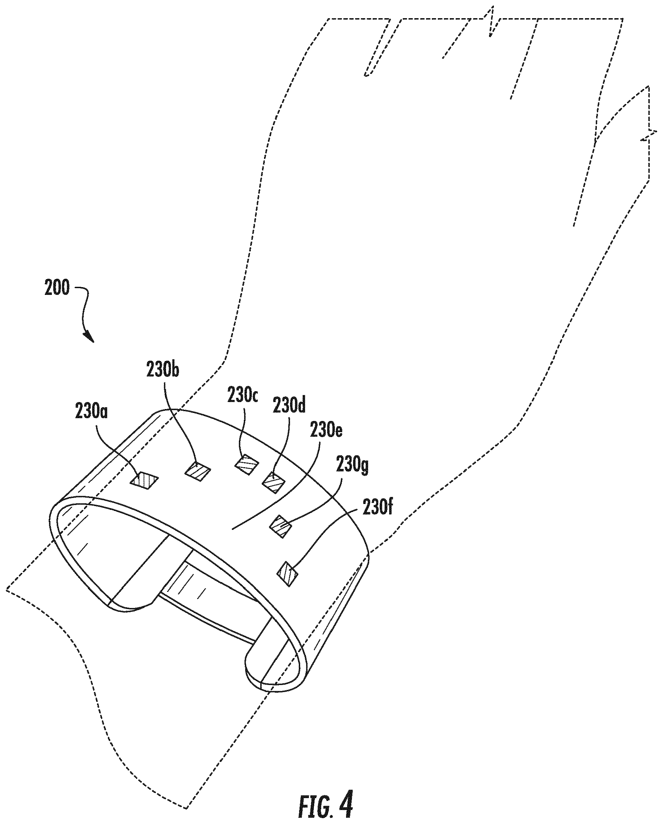

As shown in Figure 4, the nanosensor 230 is a biometric reading device. In an embodiment, the nanosensor 230 can be one or more of a bioimpedance sensor 230a, an optical heart rate sensor 230b, a galvanic skin response sensor 230c, an accelerometer 230d, a gyroscope 230e, a thermometer 230f, radiation sensor 230g, or any combination thereof. In an embodiment, the bioimpedance sensor 230a is configured to measure one or more of a user heart rate, respiration level, or hydration level. In an embodiment, the optical heart rate sensor 230b measure user heart rate by using a light sensor that detects minor fluctuations in the user's capillaries. In an embodiment, the galvanic skin response sensor 230c is configured to monitor user sweat levels by measuring electrical conductance of the user's skin. In an embodiment, the accelerometer 230d is configured to count user steps or record sudden changes in movement. In an embodiment, the gyroscope 230e is configured to measure orientation of a user. In an embodiment, thermometer 230f is configured to monitor user body temperature. In an embodiment, the radiation sensor 230g is a radiation dosimeter configured to monitor levels of user ultra-violet (UV), high-energy beta, gamma, and/or x-ray radiation exposure.

Each nanosensor 230a-g is electrically connected to the second microprocessor 205, and each sends biometric information to the microprocessor 205. The microprocessor 205 can store the biometric information in the second memory 210, and/or send the biometric information to the wireless communication system 220.

In an embodiment, the biometrics monitor 200 includes a second GPS subsystem 240. Similar to the first GPS subsystem 160, the second GPS subsystem 240 includes a GPS receiver 241, GPS antenna 242, and a GPS static memory 243. The GPS receiver 241 is electrically connected to the second microprocessor 205, GPS antenna 242, and GPS static memory 243. The GPS antenna 242 receives signals from global positioning satellites, which in turn, are then sent to the GPS receiver 241. The GPS receiver 241 sends the signals to the GPS static memory 243, which stores current and/or past positioning information and associated time stamps. The GPS receiver 241 can transmit this current and/or past positioning information and associated time stamps to the second microprocessor 205, which in turn, can send this information to be stored in the second memory 210, and/or can be sent to second low energy transceiver 221 upon PLB 100 activation. Additionally, in an embodiment the GPS static memory 243 can store route information and speed of travel for a user based on the current and past positioning information and associated time stamps, and this information can also be accessed by the second microprocessor 205.

As shown in the embodiment of Figure 1, the personal locator beacon 100 is communicatively coupled to the biometrics monitor 200. In an embodiment, the personal locator beacon 100 is communicatively coupled to the biometrics monitor 200 through the first low energy transceiver 165 and first low energy antennae 170, and the second low energy transceiver 221 and second low energy antennae 223. The biometric monitor 200 sends biometric parameters collected from the nanosensors 230a-g to the personal locator beacon 100 using low energy Bluetooth signals at configured intervals. The configured intervals can be an event triggered intervals (e.g. activation of personal locator beacon 100 to broadcast distress signal 11), predetermined fixed intervals (e.g. user configured times), profile based intervals (e.g. schedule or activity specific times), or any combination thereof.

In an embodiment, the biometrics monitor 200 sends user geographical information collected from the second GPS subsystem 240 to the personal locator beacon 100, either individually, or in combination with the biometric parameters collected from the nanosensors 230a-g. The user geographical information can be sent to the personal locator beacon 100 at the configured intervals.

In an embodiment, the biometrics monitor 200 can include an identification serial number unique to the biometric monitor 200. The identification serial number can be sent to the personal locator beacon 100 at the same or different time the biometric parameters and/or user geographical information is sent from the biometric monitor 200 to the personal locator beacon 100.

In an embodiment, the personal locator beacon system 1 can included two or more biometrics monitors 200, each of which can be worn by a different user. Each biometrics monitor 200 would include an identification serial number unique to that biometric monitor 100, and each of the biometric monitors 200 can send its identification serial number to the personal locator beacon 100 at the same or different time the biometric parameters and/or user geographical information is sent from each biometric monitor 200 to the personal locator beacon 100.

As generally shown in the embodiment of Figure 1, when the personal locator beacon 100 has been activated to broadcast a distress signal 11 using the radio frequency transmitter 115 via the first radio frequency antenna 125, the distress signal includes geographical coordinates determined by the first GPS subsystem 160. In another embodiment, the distress signal 11 would include both the geographical coordinates from the first GPS subsystem 160 and biometric parameters received from one or more nanosensors 230. In another embodiment, the distress signal 11 would include the geographical coordinates from the first GPS subsystem 160 and the user geographical coordinates received from the second GPS subsystem 240. In yet another embodiment, the distress signal 11 would include the geographical coordinates from the first GPS subsystem 160, the user geographical coordinates received from the second GPS subsystem 240, and the biometric parameters received from one or more nanosensors 230. In another embodiment, the distress signal 11 can include geographical coordinates from the first GPS subsystem 160, user geographical coordinates received from the second GPS subsystem 240, biometric parameters received from one or more nanosensors 230, identification serial number of the biometric monitor 200, or any combination thereof.

Thus, as generally shown in the embodiment of Figure 1, the biometrics monitor 200 broadcasts a biometrics containing signal 12 to the personal locator beacon 100. The personal locator beacon 100 in turn, broadcasts the distress signal 11 which is detected by either ground-based or satellite-based communication systems 13. These communication systems 13 then relay 14 the information contained in the distress signal 11 to the appropriate emergency responders 15.

By including additional geographical and biometric parameter information in the distress signal 11, emergency responders 15 will be alerted to both the exact geographical location of the user, as well as the general health condition of the user before embarking on the rescue mission. Thus, it is possible for the emergency responders 15 to gear up with emergency equipment that fits the user's situation.

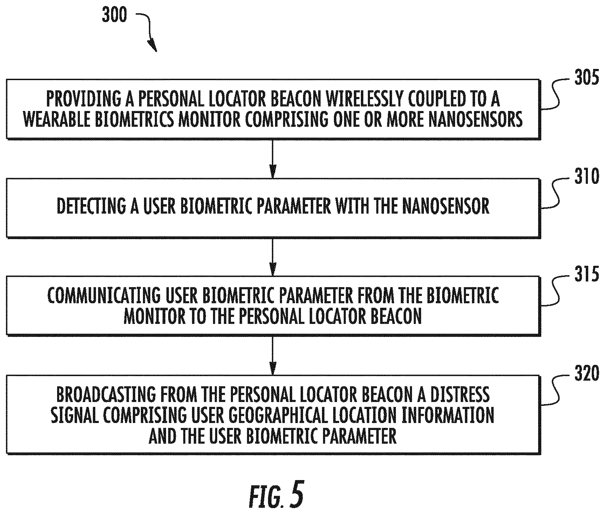

In an embodiment shown in Figure 5, a method 300 of monitoring biometrics parameters in the personal locator beacon system 1 comprises providing a personal locator beacon 100 wirelessly coupled to a wearable biometrics monitor 200 having one or more nanosensors 230 at block 305; detecting a user biometric parameter by the nanosensor 230 at block 310; communicating user biometric parameter from the biometric monitor 200 to the personal locator beacon 100 at block 315; and broadcasting from the personal locator beacon 100 a distress signal 11 comprising geographical location information and the user biometric parameter at block 320.

In an embodiment, the biometric parameters from the biometric monitor are communicated to the personal locator beacon 100 at configured intervals. The configured intervals can be event triggered intervals (e.g. when the personal locator beacon is manually or automatically activated), predetermined fixed intervals set by the user or manufacturer, user profile based intervals (e.g. based on the particular activity such as remote lone worker or adventure tourist; working environment of the user; or employer duty of care), or any combination thereof.

In an embodiment, the personal locator beacon 100 is a Cospas Sarsat distress beacon or a vehicle satcom relay.

To supplement the present disclosure, this application incorporates entirely by reference the following patents, patent application publications, and patent applications:
U.S. Patent No. 6,832,725; U.S. Patent No. 7,128,266;
U.S. Patent No. 7,159,783; U.S. Patent No. 7,413,127;
U.S. Patent No. 7,726,575; U.S. Patent No. 8,294,969;
U.S. Patent No. 8,317,105; U.S. Patent No. 8,322,622;
U.S. Patent No. 8,366,005; U.S. Patent No. 8,371,507;
U.S. Patent No. 8,376,233; U.S. Patent No. 8,381,979;
U.S. Patent No. 8,390,909; U.S. Patent No. 8,408,464;
U.S. Patent No. 8,408,468; U.S. Patent No. 8,408,469;
U.S. Patent No. 8,424,768; U.S. Patent No. 8,448,863;
U.S. Patent No. 8,457,013; U.S. Patent No. 8,459,557;
U.S. Patent No. 8,469,272; U.S. Patent No. 8,474,712;
U.S. Patent No. 8,479,992; U.S. Patent No. 8,490,877;
U.S. Patent No. 8,517,271; U.S. Patent No. 8,523,076;
U.S. Patent No. 8,528,818; U.S. Patent No. 8,544,737;
U.S. Patent No. 8,548,242; U.S. Patent No. 8,548,420;
U.S. Patent No. 8,550,335; U.S. Patent No. 8,550,354;
U.S. Patent No. 8,550,357; U.S. Patent No. 8,556,174;
U.S. Patent No. 8,556,176; U.S. Patent No. 8,556,177;
U.S. Patent No. 8,559,767; U.S. Patent No. 8,599,957;
U.S. Patent No. 8,561,895; U.S. Patent No. 8,561,903;
U.S. Patent No. 8,561,905; U.S. Patent No. 8,565,107;
U.S. Patent No. 8,571,307; U.S. Patent No. 8,579,200;
U.S. Patent No. 8,583,924; U.S. Patent No. 8,584,945;
U.S. Patent No. 8,587,595; U.S. Patent No. 8,587,697;
U.S. Patent No. 8,588,869; U.S. Patent No. 8,590,789;
U.S. Patent No. 8,596,539; U.S. Patent No. 8,596,542;
U.S. Patent No. 8,596,543; U.S. Patent No. 8,599,271;
U.S. Patent No. 8,599,957; U.S. Patent No. 8,600,158;
U.S. Patent No. 8,600,167; U.S. Patent No. 8,602,309;
U.S. Patent No. 8,608,053; U.S. Patent No. 8,608,071;
U.S. Patent No. 8,611,309; U.S. Patent No. 8,615,487;
U.S. Patent No. 8,616,454; U.S. Patent No. 8,621,123;
U.S. Patent No. 8,622,303; U.S. Patent No. 8,628,013;
U.S. Patent No. 8,628,015; U.S. Patent No. 8,628,016;
U.S. Patent No. 8,629,926; U.S. Patent No. 8,630,491;
U.S. Patent No. 8,635,309; U.S. Patent No. 8,636,200;
U.S. Patent No. 8,636,212; U.S. Patent No. 8,636,215;
U.S. Patent No. 8,636,224; U.S. Patent No. 8,638,806;
U.S. Patent No. 8,640,958; U.S. Patent No. 8,640,960;
U.S. Patent No. 8,643,717; U.S. Patent No. 8,646,692;
U.S. Patent No. 8,646,694; U.S. Patent No. 8,657,200;
U.S. Patent No. 8,659,397; U.S. Patent No. 8,668,149;
U.S. Patent No. 8,678,285; U.S. Patent No. 8,678,286;
U.S. Patent No. 8,682,077; U.S. Patent No. 8,687,282;
U.S. Patent No. 8,692,927; U.S. Patent No. 8,695,880;
U.S. Patent No. 8,698,949; U.S. Patent No. 8,717,494;
U.S. Patent No. 8,717,494; U.S. Patent No. 8,720,783;
U.S. Patent No. 8,723,804; U.S. Patent No. 8,723,904;
U.S. Patent No. 8,727,223; U.S. Patent No. D702,237;
U.S. Patent No. 8,740,082; U.S. Patent No. 8,740,085;
U.S. Patent No. 8,746,563; U.S. Patent No. 8,750,445;
U.S. Patent No. 8,752,766; U.S. Patent No. 8,756,059;
U.S. Patent No. 8,757,495; U.S. Patent No. 8,760,563;
U.S. Patent No. 8,763,909; U.S. Patent No. 8,777,108;
U.S. Patent No. 8,777,109; U.S. Patent No. 8,779,898;
U.S. Patent No. 8,781,520; U.S. Patent No. 8,783,573;
U.S. Patent No. 8,789,757; U.S. Patent No. 8,789,758;
U.S. Patent No. 8,789,759; U.S. Patent No. 8,794,520;
U.S. Patent No. 8,794,522; U.S. Patent No. 8,794,525;
U.S. Patent No. 8,794,526; U.S. Patent No. 8,798,367;
U.S. Patent No. 8,807,431; U.S. Patent No. 8,807,432;
U.S. Patent No. 8,820,630; U.S. Patent No. 8,822,848;
U.S. Patent No. 8,824,692; U.S. Patent No. 8,824,696;
U.S. Patent No. 8,842,849; U.S. Patent No. 8,844,822;
U.S. Patent No. 8,844,823; U.S. Patent No. 8,849,019;
U.S. Patent No. 8,851,383; U.S. Patent No. 8,854,633;
U.S. Patent No. 8,866,963; U.S. Patent No. 8,868,421;
U.S. Patent No. 8,868,519; U.S. Patent No. 8,868,802;
U.S. Patent No. 8,868,803; U.S. Patent No. 8,870,074;
U.S. Patent No. 8,879,639; U.S. Patent No. 8,880,426;
U.S. Patent No. 8,881,983; U.S. Patent No. 8,881,987;
U.S. Patent No. 8,903,172; U.S. Patent No. 8,908,995;
U.S. Patent No. 8,910,870; U.S. Patent No. 8,910,875;
U.S. Patent No. 8,914,290; U.S. Patent No. 8,914,788;
U.S. Patent No. 8,915,439; U.S. Patent No. 8,915,444;
U.S. Patent No. 8,916,789; U.S. Patent No. 8,918,250;
U.S. Patent No. 8,918,564; U.S. Patent No. 8,925,818;
U.S. Patent No. 8,939,374; U.S. Patent No. 8,942,480;
U.S. Patent No. 8,944,313; U.S. Patent No. 8,944,327;
U.S. Patent No. 8,944,332; U.S. Patent No. 8,950,678;
U.S. Patent No. 8,967,468; U.S. Patent No. 8,971,346;
U.S. Patent No. 8,976,030; U.S. Patent No. 8,976,368;
U.S. Patent No. 8,978,981; U.S. Patent No. 8,978,983;
U.S. Patent No. 8,978,984; U.S. Patent No. 8,985,456;
U.S. Patent No. 8,985,457; U.S. Patent No. 8,985,459;
U.S. Patent No. 8,985,461; U.S. Patent No. 8,988,578;
U.S. Patent No. 8,988,590; U.S. Patent No. 8,991,704;
U.S. Patent No. 8,996,194; U.S. Patent No. 8,996,384;
U.S. Patent No. 9,002,641; U.S. Patent No. 9,007,368;
U.S. Patent No. 9,010,641; U.S. Patent No. 9,015,513;
U.S. Patent No. 9,016,576; U.S. Patent No. 9,022,288;
U.S. Patent No. 9,030,964; U.S. Patent No. 9,033,240;
U.S. Patent No. 9,033,242; U.S. Patent No. 9,036,054;
U.S. Patent No. 9,037,344; U.S. Patent No. 9,038,911;
U.S. Patent No. 9,038,915; U.S. Patent No. 9,047,098;
U.S. Patent No. 9,047,359; U.S. Patent No. 9,047,420;
U.S. Patent No. 9,047,525; U.S. Patent No. 9,047,531;
U.S. Patent No. 9,053,055; U.S. Patent No. 9,053,378;
U.S. Patent No. 9,053,380; U.S. Patent No. 9,058,526;
U.S. Patent No. 9,064,165; U.S. Patent No. 9,064,167;
U.S. Patent No. 9,064,168; U.S. Patent No. 9,064,254;
U.S. Patent No. 9,066,032; U.S. Patent No. 9,070,032;
U.S. Design Patent No. D716,285;
U.S. Design Patent No. D723,560;
U.S. Design Patent No. D730,357;
U.S. Design Patent No. D730,901;
U.S. Design Patent No. D730,902;
U.S. Design Patent No. D733,112;
U.S. Design Patent No. D734,339;
International Publication No. 2013/163789;
International Publication No. 2013/173985;
International Publication No. 2014/019130;
International Publication No. 2014/110495;
U.S. Patent Application Publication No. 2008/0185432;
U.S. Patent Application Publication No. 2009/0134221;
U.S. Patent Application Publication No. 2010/0177080;
U.S. Patent Application Publication No. 2010/0177076;
U.S. Patent Application Publication No. 2010/0177707;
U.S. Patent Application Publication No. 2010/0177749;
U.S. Patent Application Publication No. 2010/0265880;
U.S. Patent Application Publication No. 2011/0202554;
U.S. Patent Application Publication No. 2012/0111946;
U.S. Patent Application Publication No. 2012/0168511;
U.S. Patent Application Publication No. 2012/0168512;
U.S. Patent Application Publication No. 2012/0193423;
U.S. Patent Application Publication No. 2012/0203647;
U.S. Patent Application Publication No. 2012/0223141;
U.S. Patent Application Publication No. 2012/0228382;
U.S. Patent Application Publication No. 2012/0248188;
U.S. Patent Application Publication No. 2013/0043312;
U.S. Patent Application Publication No. 2013/0082104;
U.S. Patent Application Publication No. 2013/0175341;
U.S. Patent Application Publication No. 2013/0175343;
U.S. Patent Application Publication No. 2013/0257744;
U.S. Patent Application Publication No. 2013/0257759;
U.S. Patent Application Publication No. 2013/0270346;
U.S. Patent Application Publication No. 2013/0287258;
U.S. Patent Application Publication No. 2013/0292475;
U.S. Patent Application Publication No. 2013/0292477;
U.S. Patent Application Publication No. 2013/0293539;
U.S. Patent Application Publication No. 2013/0293540;
U.S. Patent Application Publication No. 2013/0306728;
U.S. Patent Application Publication No. 2013/0306731;
U.S. Patent Application Publication No. 2013/0307964;
U.S. Patent Application Publication No. 2013/0308625;
U.S. Patent Application Publication No. 2013/0313324;
U.S. Patent Application Publication No. 2013/0313325;
U.S. Patent Application Publication No. 2013/0342717;
U.S. Patent Application Publication No. 2014/0001267;
U.S. Patent Application Publication No. 2014/0008439;
U.S. Patent Application Publication No. 2014/0025584;
U.S. Patent Application Publication No. 2014/0034734;
U.S. Patent Application Publication No. 2014/0036848;
U.S. Patent Application Publication No. 2014/0039693;
U.S. Patent Application Publication No. 2014/0042814;
U.S. Patent Application Publication No. 2014/0049120;
U.S. Patent Application Publication No. 2014/0049635;
U.S. Patent Application Publication No. 2014/0061306;
U.S. Patent Application Publication No. 2014/0063289;
U.S. Patent Application Publication No. 2014/0066136;
U.S. Patent Application Publication No. 2014/0067692;
U.S. Patent Application Publication No. 2014/0070005;
U.S. Patent Application Publication No. 2014/0071840;
U.S. Patent Application Publication No. 2014/0074746;
U.S. Patent Application Publication No. 2014/0076974;
U.S. Patent Application Publication No. 2014/0078341;
U.S. Patent Application Publication No. 2014/0078345;
U.S. Patent Application Publication No. 2014/0097249;
U.S. Patent Application Publication No. 2014/0098792;
U.S. Patent Application Publication No. 2014/0100813;
U.S. Patent Application Publication No. 2014/0103115;
U.S. Patent Application Publication No. 2014/0104413;
U.S. Patent Application Publication No. 2014/0104414;
U.S. Patent Application Publication No. 2014/0104416;
U.S. Patent Application Publication No. 2014/0104451;
U.S. Patent Application Publication No. 2014/0106594;
U.S. Patent Application Publication No. 2014/0106725;
U.S. Patent Application Publication No. 2014/0108010;
U.S. Patent Application Publication No. 2014/0108402;
U.S. Patent Application Publication No. 2014/0110485;
U.S. Patent Application Publication No. 2014/0114530;
U.S. Patent Application Publication No. 2014/0124577;
U.S. Patent Application Publication No. 2014/0124579;
U.S. Patent Application Publication No. 2014/0125842;
U.S. Patent Application Publication No. 2014/0125853;
U.S. Patent Application Publication No. 2014/0125999;
U.S. Patent Application Publication No. 2014/0129378;
U.S. Patent Application Publication No. 2014/0131438;
U.S. Patent Application Publication No. 2014/0131441;
U.S. Patent Application Publication No. 2014/0131443;
U.S. Patent Application Publication No. 2014/0131444;
U.S. Patent Application Publication No. 2014/0131445;
U.S. Patent Application Publication No. 2014/0131448;
U.S. Patent Application Publication No. 2014/0133379;
U.S. Patent Application Publication No. 2014/0136208;
U.S. Patent Application Publication No. 2014/0140585;
U.S. Patent Application Publication No. 2014/0151453;
U.S. Patent Application Publication No. 2014/0152882;
U.S. Patent Application Publication No. 2014/0158770;
U.S. Patent Application Publication No. 2014/0159869;
U.S. Patent Application Publication No. 2014/0166755;
U.S. Patent Application Publication No. 2014/0166759;
U.S. Patent Application Publication No. 2014/0168787;
U.S. Patent Application Publication No. 2014/0175165;
U.S. Patent Application Publication No. 2014/0175172;
U.S. Patent Application Publication No. 2014/0191644;
U.S. Patent Application Publication No. 2014/0191913;
U.S. Patent Application Publication No. 2014/0197238;
U.S. Patent Application Publication No. 2014/0197239;
U.S. Patent Application Publication No. 2014/0197304;
U.S. Patent Application Publication No. 2014/0214631;
U.S. Patent Application Publication No. 2014/0217166;
U.S. Patent Application Publication No. 2014/0217180;
U.S. Patent Application Publication No. 2014/0231500;
U.S. Patent Application Publication No. 2014/0232930;
U.S. Patent Application Publication No. 2014/0247315;
U.S. Patent Application Publication No. 2014/0263493;
U.S. Patent Application Publication No. 2014/0263645;
U.S. Patent Application Publication No. 2014/0267609;
U.S. Patent Application Publication No. 2014/0270196;
U.S. Patent Application Publication No. 2014/0270229;
U.S. Patent Application Publication No. 2014/0278387;
U.S. Patent Application Publication No. 2014/0278391;
U.S. Patent Application Publication No. 2014/0282210;
U.S. Patent Application Publication No. 2014/0284384;
U.S. Patent Application Publication No. 2014/0288933;
U.S. Patent Application Publication No. 2014/0297058;
U.S. Patent Application Publication No. 2014/0299665;
U.S. Patent Application Publication No. 2014/0312121;
U.S. Patent Application Publication No. 2014/0319220;
U.S. Patent Application Publication No. 2014/0319221;
U.S. Patent Application Publication No. 2014/0326787;
U.S. Patent Application Publication No. 2014/0332590;
U.S. Patent Application Publication No. 2014/0344943;
U.S. Patent Application Publication No. 2014/0346233;
U.S. Patent Application Publication No. 2014/0351317;
U.S. Patent Application Publication No. 2014/0353373;
U.S. Patent Application Publication No. 2014/0361073;
U.S. Patent Application Publication No. 2014/0361082;
U.S. Patent Application Publication No. 2014/0362184;
U.S. Patent Application Publication No. 2014/0363015;
U.S. Patent Application Publication No. 2014/0369511;
U.S. Patent Application Publication No. 2014/0374483;
U.S. Patent Application Publication No. 2014/0374485;
U.S. Patent Application Publication No. 2015/0001301;
U.S. Patent Application Publication No. 2015/0001304;
U.S. Patent Application Publication No. 2015/0003673;
U.S. Patent Application Publication No. 2015/0009338;
U.S. Patent Application Publication No. 2015/0009610;
U.S. Patent Application Publication No. 2015/0014416;
U.S. Patent Application Publication No. 2015/0021397;
U.S. Patent Application Publication No. 2015/0028102;
U.S. Patent Application Publication No. 2015/0028103;
U.S. Patent Application Publication No. 2015/0028104;
U.S. Patent Application Publication No. 2015/0029002;
U.S. Patent Application Publication No. 2015/0032709;
U.S. Patent Application Publication No. 2015/0039309;
U.S. Patent Application Publication No. 2015/0039878;
U.S. Patent Application Publication No. 2015/0040378;
U.S. Patent Application Publication No. 2015/0048168;
U.S. Patent Application Publication No. 2015/0049347;
U.S. Patent Application Publication No. 2015/0051992;
U.S. Patent Application Publication No. 2015/0053766;
U.S. Patent Application Publication No. 2015/0053768;
U.S. Patent Application Publication No. 2015/0053769;
U.S. Patent Application Publication No. 2015/0060544;
U.S. Patent Application Publication No. 2015/0062366;
U.S. Patent Application Publication No. 2015/0063215;
U.S. Patent Application Publication No. 2015/0063676;
U.S. Patent Application Publication No. 2015/0069130;
U.S. Patent Application Publication No. 2015/0071819;
U.S. Patent Application Publication No. 2015/0083800;
U.S. Patent Application Publication No. 2015/0086114;
U.S. Patent Application Publication No. 2015/0088522;
U.S. Patent Application Publication No. 2015/0096872;
U.S. Patent Application Publication No. 2015/0099557;
U.S. Patent Application Publication No. 2015/0100196;
U.S. Patent Application Publication No. 2015/0102109;
U.S. Patent Application Publication No. 2015/0115035;
U.S. Patent Application Publication No. 2015/0127791;
U.S. Patent Application Publication No. 2015/0128116;
U.S. Patent Application Publication No. 2015/0129659;
U.S. Patent Application Publication No. 2015/0133047;
U.S. Patent Application Publication No. 2015/0134470;
U.S. Patent Application Publication No. 2015/0136851;
U.S. Patent Application Publication No. 2015/0136854;
U.S. Patent Application Publication No. 2015/0142492;
U.S. Patent Application Publication No. 2015/0144692;
U.S. Patent Application Publication No. 2015/0144698;
U.S. Patent Application Publication No. 2015/0144701;
U.S. Patent Application Publication No. 2015/0149946;
U.S. Patent Application Publication No. 2015/0161429;
U.S. Patent Application Publication No. 2015/0169925;
U.S. Patent Application Publication No. 2015/0169929;
U.S. Patent Application Publication No. 2015/0178523;
U.S. Patent Application Publication No. 2015/0178534;
U.S. Patent Application Publication No. 2015/0178535;
U.S. Patent Application Publication No. 2015/0178536;
U.S. Patent Application Publication No. 2015/0178537;
U.S. Patent Application Publication No. 2015/0181093;
U.S. Patent Application Publication No. 2015/0181109;
U.S. Patent Application No. 13/367,978 for a Laser Scanning Module Employing an Elastomeric U-Hinge Based Laser Scanning Assembly, filed February 7, 2012 (Feng *et al*.);
U.S. Patent Application No. 29/458,405 for an *Electronic Device,* filed June 19, 2013 (Fitch *et al*.);
U.S. Patent Application No. 29/459,620 for an *Electronic Device Enclosure,* filed July 2, 2013 (London *et al*.);
U.S. Patent Application No. 29/468,118 for an *Electronic Device Case,* filed September 26, 2013 (Oberpriller *et al*.);
U.S. Patent Application No. 14/150,393 for *Indicia-reader Having Unitary Construction Scanner,* filed January 8, 2014 (Colavito *et al*.);
U.S. Patent Application No. 14/200,405 for *Indicia Reader for Size-Limited Applications* filed March 7, 2014 (Feng *et al*.);
U.S. Patent Application No. 14/231,898 for *Hand-Mounted Indicia-Reading Device with Finger Motion Triggering* filed April 1, 2014 (Van Horn *et al*.);
U.S. Patent Application No. 29/486,759 for an Imaging Terminal, filed April 2, 2014 (Oberpriller et al.);
U.S. Patent Application No. 14/257,364 for *Docking System and Method Using Near Field Communication* filed April 21, 2014 (Showering);
U.S. Patent Application No. 14/264,173 for *Autofocus Lens System for Indicia Readers* filed April 29, 2014 (Ackley *et al.);*
U.S. Patent Application No. 14/277, 337 *for MULTIPURPOSE OPTICAL READER, filed May 14, 2014 (Jovanovski et al.);*
U.S. Patent Application No. 14/283,282 *for TERMINAL HAVING ILLUMINATION AND FOCUS CONTROL filed May 21, 2014 (Liu et al.);*
U.S. Patent Application No. 14/327,827 for a MOBILE-PHONE ADAPTER FOR ELECTRONIC TRANSACTIONS, filed July 10, 2014 (Hejl);
U.S. Patent Application No. 14/334,934 for a SYSTEM AND METHOD FOR INDICIA VERIFICATION, filed July 18, 2014 (Hejl);
U.S. Patent Application No. 14/339,708 for LASER SCANNING CODE SYMBOL READING SYSTEM, filed July 24, 2014 (Xian et al.);
U.S. Patent Application No. 14/340,627 for an AXIALLY REINFORCED FLEXIBLE SCAN ELEMENT, filed July 25, 2014 (Rueblinger et al.);
U.S. Patent Application No. 14/446,391 for MULTIFUNCTION POINT OF SALE APPARATUS WITH OPTICAL SIGNATURE CAPTURE filed July 30, 2014 (Good et al.);
U.S. Patent Application No. 14/452,697 for INTERACTIVE INDICIA READER, filed August 6, 2014 (Todeschini);
U.S. Patent Application No. 14/453,019 for DIMENSIONING SYSTEM WITH GUIDED ALIGNMENT, filed August 6, 2014 (Li et al.);
U.S. Patent Application No. 14/462,801 for MOBILE COMPUTING DEVICE WITH DATA COGNITION SOFTWARE, filed on August 19, 2014 (Todeschini et al.);
U.S. Patent Application No. 14/483,056 for VARIABLE DEPTH OF FIELD BARCODE SCANNER filed Sep. 10, 2014 (McCloskey et al.);
U.S. Patent Application No. 14/513,808 for IDENTIFYING INVENTORY ITEMS IN A STORAGE FACILITY filed Oct. 14, 2014 (Singel et al.);
U.S. Patent Application No. 14/519,195 for HANDHELD DIMENSIONING SYSTEM WITH FEEDBACK filed Oct. 21, 2014 (Laffargue et al.);
U.S. Patent Application No. 14/519,179 for DIMENSIONING SYSTEM WITH MULTIPATH INTERFERENCE MITIGATION filed Oct. 21, 2014 (Thuries et al.);
U.S. Patent Application No. 14/519,211 for SYSTEM AND METHOD FOR DIMENSIONING filed Oct. 21, 2014 (Ackley et al.);
U.S. Patent Application No. 14/519,233 for HANDHELD DIMENSIONER WITH DATA-QUALITY INDICATION filed Oct. 21, 2014 (Laffargue et al.);
U.S. Patent Application No. 14/519,249 for HANDHELD DIMENSIONING SYSTEM WITH MEASUREMENT-CONFORMANCE FEEDBACK filed Oct. 21, 2014 (Ackley et al.);
U.S. Patent Application No. 14/527,191 for METHOD AND SYSTEM FOR RECOGNIZING SPEECH USING WILDCARDS IN AN EXPECTED RESPONSE filed Oct. 29, 2014 (Braho et al.);
U.S. Patent Application No. 14/529,563 for ADAPTABLE INTERFACE FOR A MOBILE COMPUTING DEVICE filed Oct. 31, 2014 (Schoon et al.);
U.S. Patent Application No. 14/529,857 for BARCODE READER WITH SECURITY FEATURES filed October 31, 2014 (Todeschini et al.);
U.S. Patent Application No. 14/398,542 for PORTABLE ELECTRONIC DEVICES HAVING A SEPARATE LOCATION TRIGGER UNIT FOR USE IN CONTROLLING AN APPLICATION UNIT filed November 3, 2014 (Bian et al.);
U.S. Patent Application No. 14/531,154 for DIRECTING AN INSPECTOR THROUGH AN INSPECTION filed Nov. 3, 2014 (Miller et al.);
U.S. Patent Application No. 14/533,319 for BARCODE SCANNING SYSTEM USING WEARABLE DEVICE WITH EMBEDDED CAMERA filed Nov. 5, 2014 (Todeschini);
U.S. Patent Application No. 14/535,764 for CONCATENATED EXPECTED RESPONSES FOR SPEECH RECOGNITION filed Nov. 7, 2014 (Braho et al.);
U.S. Patent Application No. 14/568,305 for AUTO-CONTRAST VIEWFINDER FOR AN INDICIA READER filed Dec. 12, 2014 (Todeschini);
U.S. Patent Application No. 14/573,022 for DYNAMIC DIAGNOSTIC INDICATOR GENERATION filed Dec. 17, 2014 (Goldsmith);
U.S. Patent Application No. 14/578,627 for SAFETY SYSTEM AND METHOD filed Dec. 22, 2014 (Ackley et al.);
U.S. Patent Application No. 14/580,262 for MEDIA GATE FOR THERMAL TRANSFER PRINTERS filed Dec. 23, 2014 (Bowles);
U.S. Patent Application No. 14/590,024 for SHELVING AND PACKAGE LOCATING SYSTEMS FOR DELIVERY VEHICLES filed January 6, 2015 (Payne);
U.S. Patent Application No. 14/596,757 for SYSTEM AND METHOD FOR DETECTING BARCODE PRINTING ERRORS filed Jan. 14, 2015 (Ackley);
U.S. Patent Application No. 14/416,147 for OPTICAL READING APPARATUS HAVING VARIABLE SETTINGS filed January 21, 2015 (Chen et al.);
U.S. Patent Application No. 14/614,706 for DEVICE FOR SUPPORTING AN ELECTRONIC TOOL ON A USER'S HAND filed Feb. 5, 2015 (Oberpriller et al.);
U.S. Patent Application No. 14/614,796 for CARGO APPORTIONMENT TECHNIQUES filed Feb. 5, 2015 (Morton et al.);
U.S. Patent Application No. 29/516,892 for TABLE COMPUTER filed Feb. 6, 2015 (Bidwell et al.);
U.S. Patent Application No. 14/619,093 for METHODS FOR TRAINING A SPEECH RECOGNITION SYSTEM filed Feb. 11, 2015 (Pecorari);
U.S. Patent Application No. 14/628,708 for DEVICE, SYSTEM, AND METHOD FOR DETERMINING THE STATUS OF CHECKOUT LANES filed Feb. 23, 2015 (Todeschini);
U.S. Patent Application No. 14/630,841 for TERMINAL INCLUDING IMAGING ASSEMBLY filed Feb. 25, 2015 (Gomez et al.);
U.S. Patent Application No. 14/635,346 for SYSTEM AND METHOD FOR RELIABLE STORE-AND-FORWARD DATA HANDLING BY ENCODED INFORMATION READING TERMINALS filed March 2, 2015 (Sevier);
U.S. Patent Application No. 29/519,017 for SCANNER filed March 2, 2015 (Zhou et al.);
U.S. Patent Application No. 14/405,278 for DESIGN PATTERN FOR SECURE STORE filed March 9, 2015 (Zhu et al.);
U.S. Patent Application No. 14/660,970 for DECODABLE INDICIA READING TERMINAL WITH COMBINED ILLUMINATION filed March 18, 2015 (Kearney et al.);
U.S. Patent Application No. 14/661,013 for REPROGRAMMING SYSTEM AND METHOD FOR DEVICES INCLUDING PROGRAMMING SYMBOL filed March 18, 2015 (Soule et al.);
U.S. Patent Application No. 14/662,922 for MULTIFUNCTION POINT OF SALE SYSTEM filed March 19, 2015 (Van Horn et al.);
U.S. Patent Application No. 14/663,638 for VEHICLE MOUNT COMPUTER WITH CONFIGURABLE IGNITION SWITCH BEHAVIOR filed March 20, 2015 (Davis et al.);
U.S. Patent Application No. 14/664,063 for METHOD AND APPLICATION FOR SCANNING A BARCODE WITH A SMART DEVICE WHILE CONTINUOUSLY RUNNING AND DISPLAYING AN APPLICATION ON THE SMART DEVICE DISPLAY filed March 20, 2015 (Todeschini);
U.S. Patent Application No. 14/669,280 for TRANSFORMING COMPONENTS OF A WEB PAGE TO VOICE PROMPTS filed March 26, 2015 (Funyak et al.);
U.S. Patent Application No. 14/674,329 for AIMER FOR BARCODE SCANNING filed March 31, 2015 (Bidwell);
U.S. Patent Application No. 14/676,109 for INDICIA READER filed April 1, 2015 (Huck);
U.S. Patent Application No. 14/676,327 for DEVICE MANAGEMENT PROXY FOR SECURE DEVICES filed April 1, 2015 (Yeakley et al.);
U.S. Patent Application No. 14/676,898 for NAVIGATION SYSTEM CONFIGURED TO INTEGRATE MOTION SENSING DEVICE INPUTS filed April 2, 2015 (Showering);
U.S. Patent Application No. 14/679,275 for DIMENSIONING SYSTEM CALIBRATION SYSTEMS AND METHODS filed April 6, 2015 (Laffargue et al.);
U.S. Patent Application No. 29/523,098 for HANDLE FOR A TABLET COMPUTER filed April 7, 2015 (Bidwell et al.);
U.S. Patent Application No. 14/682,615 for SYSTEM AND METHOD FOR POWER MANAGEMENT OF MOBILE DEVICES filed April 9, 2015 (Murawski et al.);
U.S. Patent Application No. 14/686,822 for MULTIPLE PLATFORM SUPPORT SYSTEM AND METHOD filed April 15, 2015 (Qu et al.);
U.S. Patent Application No. 14/687,289 for SYSTEM FOR COMMUNICATION VIA A PERIPHERAL HUB filed April 15, 2015 (Kohtz et al.);
U.S. Patent Application No. 29/524,186 for SCANNER filed April 17, 2015 (Zhou et al.);
U.S. Patent Application No. 14/695,364 for MEDICATION MANAGEMENT SYSTEM filed April 24, 2015 (Sewell et al.);
U.S. Patent Application No. 14/695,923 for SECURE UNATTENDED NETWORK AUTHENTICATION filed April 24, 2015 (Kubler et al.);
U.S. Patent Application No. 29/525,068 for TABLET COMPUTER WITH REMOVABLE SCANNING DEVICE filed April 27, 2015 (Schulte et al.);
U.S. Patent Application No. 14/699,436 for SYMBOL READING SYSTEM HAVING PREDICTIVE DIAGNOSTICS filed April 29, 2015 (Nahill et al.);
U.S. Patent Application No. 14/702,110 for SYSTEM AND METHOD FOR REGULATING BARCODE DATA INJECTION INTO A RUNNING APPLICATION ON A SMART DEVICE filed May 1, 2015 (Todeschini et al.);
U.S. Patent Application No. 14/702,979 for TRACKING BATTERY CONDITIONS filed May 4, 2015 (Young et al.);
U.S. Patent Application No. 14/704,050 for INTERMEDIATE LINEAR POSITIONING filed May 5, 2015 (Charpentier et al.);
U.S. Patent Application No. 14/705,012 for HANDS-FREE HUMAN MACHINE INTERFACE RESPONSIVE TO A DRIVER OF A VEHICLE filed May 6, 2015 (Fitch et al.);
U.S. Patent Application No. 14/705,407 for METHOD AND SYSTEM TO PROTECT SOFTWARE-BASED NETWORK-CONNECTED DEVICES FROM ADVANCED PERSISTENT THREAT filed May 6, 2015 (Hussey et al.);
U.S. Patent Application No. 14/707,037 for SYSTEM AND METHOD FOR DISPLAY OF INFORMATION USING A VEHICLE-MOUNT COMPUTER filed May 8, 2015 (Chamberlin);
U.S. Patent Application No. 14/707,123 for APPLICATION INDEPENDENT DEX/UCS INTERFACE filed May 8, 2015 (Pape);
U.S. Patent Application No. 14/707,492 for METHOD AND APPARATUS FOR READING OPTICAL INDICIA USING A PLURALITY OF DATA SOURCES filed May 8, 2015 (Smith et al.);
U.S. Patent Application No. 14/710,666 for PRE-PAID USAGE SYSTEM FOR ENCODED INFORMATION READING TERMINALS filed May 13, 2015 (Smith);
U.S. Patent Application No. 29/526,918 for CHARGING BASE filed May 14, 2015 (Fitch et al.);
U.S. Patent Application No. 14/715,672 for AUGUMENTED REALITY ENABLED HAZARD DISPLAY filed May 19, 2015 (Venkatesha et al.);
U.S. Patent Application No. 14/715,916 for EVALUATING IMAGE VALUES filed May 19, 2015 (Ackley);
U.S. Patent Application No. 14/722,608 for INTERACTIVE USER INTERFACE FOR CAPTURING A DOCUMENT IN AN IMAGE SIGNAL filed May 27, 2015 (Showering et al.);
U.S. Patent Application No. 29/528,165 for IN-COUNTER BARCODE SCANNER filed May 27, 2015 (Oberpriller et al.);
U.S. Patent Application No. 14/724,134 for ELECTRONIC DEVICE WITH WIRELESS PATH SELECTION CAPABILITY filed May 28, 2015 (Wang et al.);
U.S. Patent Application No. 14/724,849 for METHOD OF PROGRAMMING THE DEFAULT CABLE INTERFACE SOFTWARE IN AN INDICIA READING DEVICE filed May 29, 2015 (Barten);
U.S. Patent Application No. 14/724,908 for IMAGING APPARATUS HAVING IMAGING ASSEMBLY filed May 29, 2015 (Barber et al.);
U.S. Patent Application No. 14/725,352 for APPARATUS AND METHODS FOR MONITORING ONE OR MORE PORTABLE DATA TERMINALS (Caballero et al.);
U.S. Patent Application No. 29/528,590 for ELECTRONIC DEVICE filed May 29, 2015 (Fitch et al.);
U.S. Patent Application No. 29/528,890 for MOBILE COMPUTER HOUSING filed June 2, 2015 (Fitch et al.);
U.S. Patent Application No. 14/728,397 for DEVICE MANAGEMENT USING VIRTUAL INTERFACES CROSS-REFERENCE TO RELATED APPLICATIONS filed June 2, 2015 (Caballero);
U.S. Patent Application No. 14/732,870 for DATA COLLECTION MODULE AND SYSTEM filed June 8, 2015 (Powilleit);
U.S. Patent Application No. 29/529,441 for INDICIA READING DEVICE filed June 8, 2015 (Zhou et al.);
U.S. Patent Application No. 14/735,717 for INDICIA-READING SYSTEMS HAVING AN INTERFACE WITH A USER'S NERVOUS SYSTEM filed June 10, 2015 (Todeschini);
U.S. Patent Application No. 14/738,038 for METHOD OF AND SYSTEM FOR DETECTING OBJECT WEIGHING INTERFERENCES filed June 12, 2015 (Amundsen et al.);
U.S. Patent Application No. 14/740,320 for TACTILE SWITCH FOR A MOBILE ELECTRONIC DEVICE filed June 16, 2015 (Bandringa);
U.S. Patent Application No. 14/740,373 for CALIBRATING A VOLUME DIMENSIONER filed June 16, 2015 (Ackley et al.);
U.S. Patent Application No. 14/742,818 for INDICIA READING SYSTEM EMPLOYING DIGITAL GAIN CONTROL filed June 18, 2015 (Xian et al.);
U.S. Patent Application No. 14/743,257 for WIRELESS MESH POINT PORTABLE DATA TERMINAL filed June 18, 2015 (Wang et al.);
U.S. Patent Application No. 29/530,600 for CYCLONE filed June 18, 2015 (Vargo et al);
U.S. Patent Application No. 14/744,633 for IMAGING APPARATUS COMPRISING IMAGE SENSOR ARRAY HAVING SHARED GLOBAL SHUTTER CIRCUITRY filed June 19, 2015 (Wang);
U.S. Patent Application No. 14/744,836 for CLOUD-BASED SYSTEM FOR READING OF DECODABLE INDICIA filed June 19, 2015 (Todeschini et al.);
U.S. Patent Application No. 14/745,006 for SELECTIVE OUTPUT OF DECODED MESSAGE DATA filed June 19, 2015 (Todeschini et al.);
U.S. Patent Application No. 14/747,197 for OPTICAL PATTERN PROJECTOR filed June 23, 2015 (Thuries et al.);
U.S. Patent Application No. 14/747,490 for DUAL-PROJECTOR THREE-DIMENSIONAL SCANNER filed June 23, 2015 (Jovanovski et al.); and
U.S. Patent Application No. 14/748,446 for CORDLESS INDICIA READER WITH A MULTIFUNCTION COIL FOR WIRELESS CHARGING AND EAS DEACTIVATION, filed June 24, 2015 (Xie et al.).

While there is shown and described herein certain exemplary embodiments of a monitoring user biometric parameters with nanotechnology in personal locator beacons, it will be manifest to those of ordinary skill in the art that various modifications and rearrangements of the parts may be made without departing from the spirit and scope of the underlying inventive concept and that the same is not limited to the particular forms herein shown and described except insofar as indicated by the scope of the appended claims.

## Claims

1. A personal locator beacon system comprising:
a personal locator beacon having
a first microprocessor,
a first global positioning subsystem coupled to the first microprocessor,
a first low energy transceiver coupled to the first microprocessor, and
a first low energy antennae coupled to the first low energy transceiver; and
a biometrics monitor having
a second microprocessor,
a second low energy transceiver coupled to the second microprocessor,
a second low energy antennae coupled to the second low energy transceiver, and
one or more nanosensors.

2. The personal locator beacon system of claim 1, wherein the first low energy transceiver, the first low energy antennae, the second low energy transceiver, and the second low energy antennae are low energy Bluetooth components.

3. The personal locator beacon system of claim 1, wherein the nanosensor is a bioimpedance sensor configured to measure one or more of a user heart rate, respiration level, or hydration level.

4. The personal locator beacon system of claim 1, wherein the nanosensor is an optical heart rate sensor.

5. The personal locator beacon system of claim 1, wherein the nanosensor is a galvanic skin response sensor configured to monitor user sweat levels.

6. The personal locator beacon system of claim 1, wherein the nanosensor is an accelerometer configured to count user steps or record sudden changes in movement.

7. The personal locator beacon system of claim 1, wherein the nanosensor is a radiation sensor configured to measure user radiation exposure.

8. The personal locator beacon system of claim 1, wherein:
the biometrics monitor comprises a second GPS subsystem coupled to the second microprocessor; and
each of the first and second GPS subsystems comprises:
a GPS receiver,
a GPS antenna, and
a GPS static memory communicatively coupled to the GPS receiver and configured to store:
positioning information,
time stamps associated with the positioning information,
route information,
speed of travel,
or any combination thereof.

9. A method of monitoring user biometric parameters in a personal locator beacon system, comprising:
providing a personal locator beacon wirelessly coupled to a wearable biometrics monitor comprising one or more nanosensors;
detecting a user biometric parameter of a user by the nanosensor;
communicating the user biometric parameter from the biometric monitor to the personal locator beacon; and
broadcasting from the personal locator beacon a distress signal comprising geographical location information and the user biometric parameter.

10. The method of claim 9, wherein the personal locator beacon comprises a GPS subsystem having:
a GPS receiver,
a GPS antenna, and
a static memory coupled to the GPS receiver and configured to store positioning information and associated time stamps.

11. The method of claim 9, wherein the nanosensor comprises:
a bioimpedance sensor configured to measure one or more user biometric parameters of a user heart rate, respiration level, or hydration level;
an optical heart rate sensor configured to measure a user biometric parameter of a user heart rate;
a galvanic skin response sensor configured to monitor a user biometric parameter of user sweat level;
an accelerometer configured to measure a user biometric parameter of user steps or sudden changes in user movement;
a gyroscope configured to measure a user biometric parameter of a user orientation;
a thermometer configured to monitor a user biometric parameter of user body temperature;
a radiation sensor configured to monitor user radiation exposure levels; or
any combination thereof.

12. The method of claim 9, wherein the biometric monitor is a wearable device comprising a glove, a wristband, a necklace, a headband, a hat, smartphone, smartwatch, or a chest strap.

13. The method of claim 9, wherein the user biometric parameters from the biometric monitor are communicated to the personal locator beacon at configured intervals.

14. The method of claim 13, wherein the configured intervals are event triggered intervals, predetermined fixed intervals, profile based intervals, or any combination thereof.

15. The method of claim 9, wherein the personal locator beacon is a Cospas-Sarsat distress beacon or a vehicle satcom relay.
